# FASCICULE DE BREVET EUROPEEN

(11) **EP 3 592 175 B1**
(45) Date de publication et mention de la délivrance du brevet: **05.05.2021**
(21) Numéro de dépôt: 18715199.8
(22) Date de dépôt: 06.03.2018
(51) Int. Cl.: A45D 34/00

(54) **ENSEMBLE DE DISTRIBUTION ET D'APPLICATION DE PRODUIT FLUIDE**
VORRICHTUNG ZUR AUSGABE UND AUFTRAGEN EINES FLUIDPRODUKTS
DEVICE FOR DISPENSING AND APPLYING A FLUID PRODUCT

(30) Priorité: 09.03.2017 FR 1751930
(43) Date de publication de la demande: 15.01.2020
(73) Titulaire: APTAR France SAS, 27110 Le Neubourg (FR)
(72) Inventeur: JEANNIN, Julien, 27400 Louviers (FR); LEBOUCHER, Arnaud, 27170 Ecardenville La Campagne (FR)
(74) Mandataire: CAPRI
(86) Numéro de dépôt international: PCT/FR2018/050510
(87) Numéro de publication internationale: WO 2018/162837

(56) Documents cités:
- EP-A1- 1 273 245
- EP-A1- 1 535 532
- WO-A1-2015/170048
- US-A1- 2005 042 020
- US-A1- 2007 274 764
- US-A1- 2016 135 567

## Description

La présente invention concerne un ensemble de distribution et d'application de produit fluide comprenant un corps de réception creux, une surface d'application de produit fluide montée à une extrémité supérieure du corps de réception et destinée à venir en contact avec une cible, telle que la peau, et un distributeur de produit fluide comprenant un réservoir, une pompe et un embout de distribution définissant une surface de sortie dans laquelle débouche un orifice de distribution de produit fluide. L'ensemble comprend avantageusement un module d'activation qui émet des ondes, de la lumière et/ou un courant électrique et les (la ou le) transmet à la surface d'application. Ainsi l'ensemble permet d'appliquer un produit fluide sur une cible telle que la peau, et simultanément, postérieurement ou antérieurement soumettre la peau à une activation au travers de la surface d'application. Le domaine d'application privilégié de la présente invention est bien entendu celui de la cosmétique, mais peut aussi être celui de la pharmacie.

Dans l'art antérieur on connait déjà le document WO2015/170048 qui décrit un ensemble de distribution et d'application de ce type. Il comprend un corps de réception creux et une surface d'application de produit fluide située à une extrémité supérieure du corps de réception et comprenant un logement. Cette surface d'application est destinée à venir en contact avec une cible, telle que la peau, cette surface d'application de produit fluide comprenant un logement. L'ensemble de distribution et d'application comprend également un distributeur de produit fluide ayant un réservoir, une pompe et un embout de distribution définissant un orifice de distribution de produit fluide. Le distributeur est reçu de manière amovible dans le corps de réception avec un appendice d'insertion de son embout de distribution inséré de manière amovible dans le logement, de sorte que l'orifice de distribution débouche directement dans la surface d'application. Le distributeur est extractible hors du corps de réception à travers une extrémité inférieure ouverte du corps de réception qui est opposée à l'extrémité supérieure. D'autre part, des moyens d'orientation angulaire sont prévus pour amener le distributeur de produit fluide dans une position angulaire déterminée par rapport au corps de réception. Plus précisément, l'embout de distribution et le logement présentent une section transversale correspondante imposant un engagement mutuel avec une orientation angulaire unique. Ainsi, pour faire pénétrer l'embout de distribution dans son logement, il faut que l'utilisateur tourne le distributeur dans le corps de réception jusqu'à ce que l'embout soit aligné avec le logement. Cela est d'autant plus fastidieux que le distributeur est déjà presque entièrement reçu dans le corps de réception.

On connaît également le document US2011/0190672 décrivant un applicateur vibrant comprenant un boîtier renfermant un réservoir pourvu d'un embout de distribution. Un collier disposé sur le boîtier renferme un élément vibrant qui entoure l'embout de distribution. Une tête de stockage thermique est couplée à l'élément vibrant pour la faire vibrer : la tête est mobile par rapport au boîtier. Cette tête définit une surface d'application dans laquelle débouche l'embout de distribution. Le collier comprend un poussoir latéral pour activer l'élément vibrant. La distribution du produit fluide s'effectue en écrasant le réservoir : une variante avec une pompe est évoquée sans rentrer dans le détail. Ce distributeur n'a pour vocation a été démonté et constitue ainsi un ensemble unitaire.

La présente invention, qui est définie par les caractéristiques de la revendication 1, a pour but d'améliorer plus particulièrement l'ensemble de distribution et d'application de produit fluide du document WO2015/170048 en facilitant l'insertion orientée du distributeur dans le corps de réception. En d'autres termes, un but de l'invention est d'améliorer les moyens d'orientation angulaire.

Pour ce faire, la présente invention propose que les moyens d'orientation angulaire induisent un mouvement de rotation du distributeur à mesure qu'il est poussé dans le corps de réception, jusqu'à ce qu'il atteigne sa position angulaire déterminée. Ainsi, l'utilisateur n'a plus besoin de faire tourner le distributeur dans le corps de réception et de chercher à tâtonnement l'orientation angulaire précise qui permet d'engager à fond l'embout de distribution dans son logement. L'orientation adéquate du distributeur dans le corps de réception se fait maintenant automatiquement : il suffit à l'utilisateur de pousser axialement le distributeur dans le corps de réception. Au mieux, la poussée axiale ne nécessite aucune composante rotative et au pire, l'utilisateur accompagne le distributeur dans le mouvement de rotation induit par les moyens d'orientation angulaire. Il est à noter que l'orientation angulaire finale est atteinte, quelle que soit l'orientation angulaire initiale.

Selon un mode de réalisation avantageux, les moyens d'orientation angulaire comprennent au moins une rampe de came hélicoïdale. Cette came peut être formée par le distributeur ou par le corps de réception. Avantageusement, les moyens d'orientation angulaire comprennent un ergot qui coulisse le long de la rampe de came hélicoïdale en induisant un mouvement de rotation au distributeur. Par « ergot », il faut entendre tout organe, pièce ou profil susceptible de se déplacer en contact coulissant le long de la rampe ou came. De préférence, les moyens d'orientation angulaire comprennent deux rampes de came hélicoïdales qui se rejoignent au niveau d'une pointe et qui conduisent tous deux vers la position angulaire déterminée. Ainsi, le distributeur tournera dans le sens dextrogyre ou lévogyre selon que l'ergot s'engagera sur une rampe ou l'autre. Quoi qu'il arrive, l'ergot atteindra la même position. Quant à la pointe formée aux sommets des deux rampes, elle permet d'éviter le blocage de l'ergot.

Selon un mode de réalisation pratique préféré, l'embout de distribution forme, en amont de l'appendice d'insertion, deux rampes de came hélicoïdales qui se rejoignent au niveau d'une pointe et qui conduisent tous deux vers une encoche de butée, le corps de réception formant un ergot fixe qui coulisse le long d'une des deux rampes de came hélicoïdales jusqu'à venir se loger dans l'encoche de butée.

Selon un autre aspect de l'invention, l'ensemble de distribution et d'application peut comprendre un fond amovible qui coopère avec le corps de réception pour obturer l'extrémité inférieure, ce fond amovible poussant le distributeur vers l'extrémité supérieure en appuyant l'ergot fixe dans l'encoche de butée. On peut même se servir du fond pour déplacer axialement le distributeur dans le corps de réception et induire sa rotation.

Selon autre caractéristique avantageuse, le distributeur définit un axe longitudinal X qui passe par le réservoir, la pompe et l'embout de distribution, la pompe comprenant un actionneur latéral qui est mobile transversalement à l'axe longitudinal X, le corps de réception comprenant un poussoir latéral qui est disposé en face de l'actionneur latéral pour pourvoir déplacer l'actionneur latéral en appuyant latéralement sur le poussoir latéral. Grâce aux moyens d'orientation angulaire de l'invention, on garantit que l'actionneur latéral est toujours positionné au niveau du poussoir latéral. L'utilisateur n'a même plus besoin de s'en soucier, étant donné que l'orientation correcte du distributeur dans le corps de réception est désormais effectuée automatiquement.

Les moyens d'orientation angulaire de l'invention génèrent encore un autre avantage, à savoir que l'appendice d'insertion peut présenter une section transversale sensiblement ou parfaitement circulaire. Ce n'est pas le cas dans le document WO2015/170048 où l'embout de distribution et le logement présentent une section transversale correspondante imposant un engagement mutuel avec une orientation angulaire unique. Avec l'invention, la section transversale correspondante de l'embout de distribution et du logement peuvent être quelconque.

Selon un autre aspect avantageux de l'invention, l'embout de distribution définit une surface de sortie qui présente un contour extérieur dont la forme correspond à celle du logement au moins au niveau de la surface d'application, de sorte que l'embout de distribution obture le logement de manière étanche, au moins au niveau de la surface d'application. Ainsi, il ne peut y avoir de produit fluide qu'au niveau de la surface d'application.

Selon une mise en œuvre avantageuse de l'invention, le corps de réception abrite un module d'activation apte à produire des ondes électromagnétiques telles que de la lumière, des vibrations et/ou des courants électriques tels que l'iontophorèse, au niveau de la surface d'application.

L'esprit de l'invention réside dans le fait que l'orientation angulaire du distributeur dans le corps de réception s'effectue automatiquement, voire même imperceptiblement, à partir d'une position angulaire quelconque, avec l'insertion axiale du distributeur dans le corps de réception, et ce, grâce aux moyens d'orientation angulaire de l'invention. Cela permet d'orienter correctement l'embout de distribution dans son logement et/ou l'actionneur latéral en face du poussoir latéral. Ces moyens d'orientation angulaire permettent de transformer une composante axiale en une composante radiale ou rotative : une technique préférée met en œuvre une came ou rampe hélicoïdale sur laquelle coulisse un élément ou profil quelconque.

L'invention sera maintenant décrite plus en détail en référence aux dessins joints, donnant à titre d'exemple non limitatif, un mode de réalisation de l'invention.

Sur les figures :
La figure 1 est une vue en section transversale verticale à travers un ensemble de distribution et d'application de produit fluide de l'art antérieur,
La figure 2 est une vue fortement agrandie de la partie supérieure de la figure 1,
La figure 3 est une vue en section transversale horizontale le long de la ligne de section A-A de la figure 2,
La figure 4 est une vue en section transversale verticale à travers un distributeur de produit fluide selon l'invention,
Les figures 5a et 5b sont des vues en perspective sous des angles différents du distributeur de produit fluide de la figure 4,
Les figures 6a et 6b sont des vues en perspective sous des angles différents de l'embout de distribution du distributeur de produit fluide des figures 4, 5a et 5b, et
Les figures 7a, 7b et 7c sont des vues en perspective partiellement découpées d'un ensemble de distribution et d'application de produit fluide selon l'invention mettant en œuvre le distributeur de produit fluide des figures 4, 5a et 5b, dans le but d'illustrer différentes étapes lors de l'insertion du distributeur.

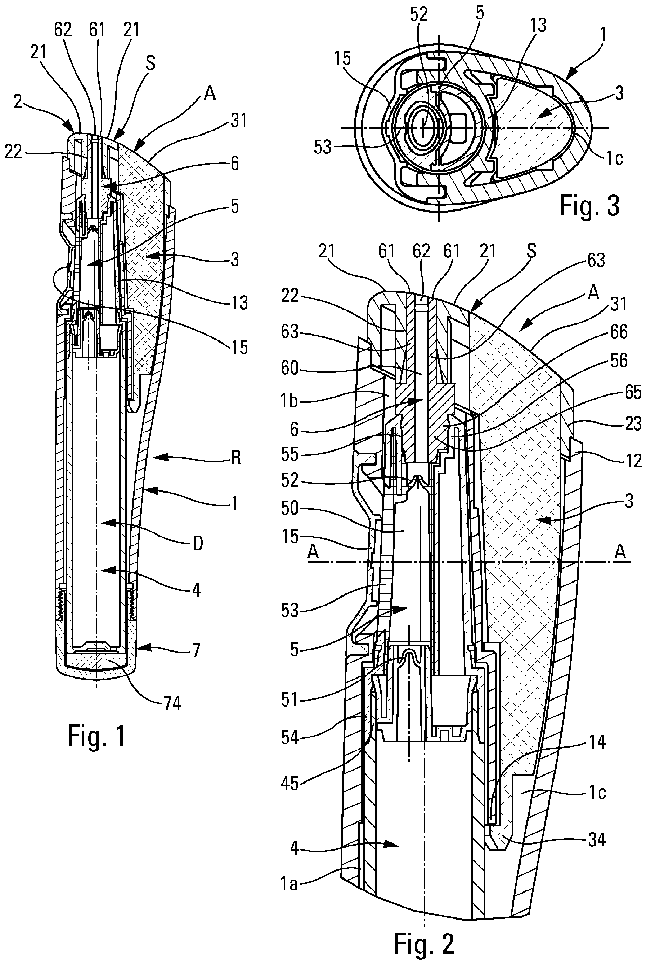

Comme susmentionné, les figures 1 à 3 représentent un ensemble de distribution et d'application de produit fluide de l'art antérieur, et en l'occurrence celui du document WO2015/170048. L'architecture globale, et même la plupart des détails, de cet ensemble de distribution et d'application de l'art antérieur, peut être reprise approximativement, voire fidèlement, dans le cadre de la présente invention, hormis les moyens d'orientation ou d'indexation du distributeur dans le corps de réception.

Cet ensemble de distribution et d'application de l'art antérieur présente une forme allongée ou élancée qui peut s'apparenter à celle d'un stylo ou d'un feutre. On peut également noter que sa section transversale n'est pas constante, puisqu'elle varie de bas en haut de manière significative. En l'occurrence, à proximité de son extrémité inférieure, l'ensemble de distribution et d'application présente une section transversale plutôt ronde ou circulaire, alors qu'au niveau de la ligne de coupe A-A, qui est plutôt située à proximité de l'extrémité supérieure, l'ensemble de distribution et d'application présente une section transversale de forme ovoïdale (Figure 3). La face supérieure de l'ensemble forme une surface d'application S qui présente une inclinaison ou pente vers un côté.

En se référant à la figure 1, on peut voir les différents éléments constitutifs de l'ensemble de distribution et d'application de l'invention. On peut tout d'abord remarquer qu'il comprend trois entités principales distinctes, à savoir un distributeur de produit fluide D, une entité de réception R et une entité d'application A. Le distributeur D est reçu de manière amovible à l'intérieur de l'entité de réception R, qui comprend un corps de réception 1 dont l'intérieur est creux. L'entité d'application A est montée sur et dans le corps 1, avantageusement de manière amovible. Ainsi de manière préférentielle, les deux entités D et A sont reçues de manière amovible sur et dans le corps 1 à partir de ces deux extrémités opposées 17 et 12, respectivement. Il s'agit là de l'architecture globale de cet ensemble de distribution et d'application de l'art antérieur, qui peut être reprise dans le cadre de l'invention.

Plus en détail, le corps 1 de l'entité de réception R est ouvert à ses deux extrémités haute 12 et basse 17 pour pouvoir accueillir les entités A et D. L'extrémité basse 17 est avantageusement filetée pour recevoir par vissage un fond amovible 7. On peut remarquer que le fond amovible 7 est pourvu d'une pièce de matière élastique 74, qui peut être une mousse ou un élastomère. Le fond amovible 7 forme intérieurement un espace qui communique vers le haut avec l'intérieur du corps 1 qui définit lui-même un espace de réception 1a. Au-delà de cet espace de réception 1a, l'intérieur du corps 1 est divisé en deux compartiments 1b et 1c par une cloison de séparation 13. Le compartiment 1b s'étend axialement dans le prolongement de l'espace 1a, alors que le compartiment 1c s'étend latéralement, au niveau où le corps 1 définit sa forme ovoïdale. L'extrémité inférieure de la cloison 13 forme une arête d'encliquetage 14 comme on le verra ci après. Le corps 1, au niveau du compartiment 1b est pourvu d'un poussoir latéral 15 qui est déplaçable transversalement à l'axe longitudinal du corps de réception 1. Le déplacement du poussoir 15 peut se faire par translation pure ou par déformation élastique. On peut par exemple prévoir de surmouler le poussoir 15 sur le corps de réception 1 avec une matière élastomérique. En variante, on peut également prévoir un poussoir 15 qui se déplace totalement indépendamment du corps 1. On peut aussi prévoir une simple fenêtre à la place du poussoir 15. On peut également remarquer que la cloison de séparation 13 s'étend jusqu'à proximité de l'extrémité haute 12. Le corps de réception 1 peut très simplement être réalisé par injection moulage de matière plastique, ou même de métal.

L'entité d'application A résulte ici de l'association d'une tête d'application 2 et d'un module d'activation 3. La tête d'application 2, comme on peut le voir sur les figures 1 et 2, comprend un logement axial 22 formé par une tubulure cylindrique dont la section transversale présente une forme géométrique complexe, par exemple celle d'un croissant. Ce logement 22 se raccorde vers le haut à une plage de surface d'application 21 qui est ici formée avec deux ouvertures, à savoir une première ouverture correspondant à l'embouchure du logement 22 et une seconde ouverture pour le module d'activation 3. Plus précisément, le module d'activation 3 comprend une section de surface d'application 31 qui obture l'ouverture correspondante de la tête 2 de manière à compléter de manière continue et lisse la plage de surface d'application 21 de la tête 2. En d'autres termes, le module d'activation 3 s'adapte dans l'ouverture de la tête d'application de sorte que la section de surface d'application 31 du module d'activation 3 complète la plage de surface d'application de la tête 2 sans créer de discontinuité, de saillant ou de creux. Par conséquent, l'assemblage du module 3 et de la tête 2 permet de créer une surface d'application S dont la seule ouverture, à ce stade, est formée par l'embouchure du logement 22. On peut remarquer sur les figures 1 et 2 que la section de surface d'application 31 s'étend au niveau de la partie la plus inclinée de la surface d'application S. La tête d'application 2 comprend également une jupe périphérique 23 qui vient s'adapter dans l'extrémité haute 12 du corps creux 1. D'autre part, le module de génération d'ondes 3 s'étend à l'intérieur de l'espace de réception 1c, et présente avantageusement un profil d'encliquetage 34 apte à coopérer avec le bord inférieur 14 de la cloison de séparation 13. De cette manière, l'entité d'application A peut être montée de manière parfaitement stable sur et dans el corps creux 1.

Selon l'invention, le module d'activation 3 permet de générer tout type d'ondes ou de rayonnement électromagnétique, de vibrations, de courants électriques, etc., comme par exemple de la lumière visible, des infrarouges, des ultraviolets, voire des micro-ondes, etc. ou encore des ultrasons ou des vibrations mécaniques. Le module 3 peut également générer de la chaleur ou du froid (ondes thermiques) pour conférer un effet chaud ou froid au contact avec la peau. L'iontophorèse est aussi une technologie qui peut être intégrée au module d'activation 3.

Le distributeur de produit fluide D comprend un réservoir de produit fluide 4, une pompe 5 et un embout de distribution 6, comme on peut le voir sur les figures 1 et 3.

Le réservoir 4 peut par exemple se présenter sous la forme d'un fût de coulissement dans lequel est reçu un piston suiveur qui est adapté à coulisser dans le fût à mesure que du produit fluide est extrait du réservoir. Le sommet du fût forme un col 45. A la place de ce réservoir particulier, on peut également imaginer un réservoir plus simple sans variation de volume utile, ou encore un réservoir avec une poche souple.

La pompe 5 comprend une bague de fixation 54 permettant son montage sur le col 45 du réservoir 4. La pompe 5 comprend une chambre de pompe 50 pourvue à son extrémité inférieure d'un clapet d'entrée 51, par exemple sous la forme d'un obturateur à fente. A son extrémité supérieure, la chambre de pompe 50 comprend un clapet de sortie 52, qui peut par exemple être également réalisé sous la forme d'un obturateur à fente. En outre, la chambre de pompe 50 comprend un actionneur latéral 53 qui permet de réduire le volume utile de la chambre de pompe 50 et ainsi de refouler du produit fluide à travers le clapet de sortie 52. L'actionneur latéral 53 est déplaçable perpendiculairement à l'axe longitudinal X du distributeur D. Le déplacement peut se faire translativement ou encore par déformation élastique. Dans le mode de réalisation utilisé pour illustrer la présente invention, l'actionneur 53 se présente sous la forme d'une paroi souple de la chambre de pompe 50 qui a été réalisée par exemple par un procédé de bi-injection ou de surmoulage. La pompe 5 peut ainsi être qualifiée de pompe à membrane souple, en ce sens que l'on agit directement sur une paroi mobile de la chambre pour mettre le produit fluide sous pression. A son extrémité supérieure, la pompe 5 forme un puits de montage 55 pour l'embout de distribution 6. Ce puits de montage 55 est avantageusement pourvu de moyens de détrompage 56, par exemple sous la forme d'un profil saillant ou d'un évidement, permettant d'imposer l'orientation angulaire de l'embout 6 dans le puits 55.

L'embout de distribution 6 comprend ainsi un talon de montage 65 qui est engagé, et avantageusement encliqueté, à l'intérieur du puits de montage 55. Le talon de montage 65 comprend un profil de détrompage 66 qui s'adapte parfaitement dans les moyens de détrompage 56 du puits 55, afin d'imposer l'orientation angulaire de l'embout de distribution 6 sur la pompe 5. De cette manière, l'embout est toujours orienté de la même manière par rapport à l'actionneur latéral 53, qui ne s'étend que sur un côté de la pompe 5. Au-dessus de ce talon de montage 65, l'embout de distribution 6 forme un appendice d'insertion 63 dont la section transversale présente une forme correspondante à celle du logement 22 formé par la tête d'application 2. Elle s'apparente à une forme d'un croissant. La paroi latérale de l'appendice d'insertion 63 peut être cylindrique, bien que non circulaire sur toute sa hauteur. A son extrémité supérieure, l'appendice 63 forme une surface de sortie sensiblement plane 61 qui est percée d'un orifice de distribution 62, formant la sortie d'un conduit de sortie 60 qui traverse l'appendice 63 et le talon de montage 65, comme on peut clairement le voir sur la figure 2.

Une fois l'embout de distribution 6 monté sur la pompe 5, comme visible sur la figure 2, on peut voir que le clapet de sortie 51 communique directement avec le conduit de sortie 60. Ainsi, en enfonçant l'actionneur latéral 53, on réduit le volume utile de la chambre de pompe 50, et du produit fluide sous pression est refoulé à travers le clapet de sortie 52, d'où il peut s'écouler à travers le conduit de sortie 60 jusqu'à l'orifice de distribution 62 situé au niveau de la surface de sortie 61. Dès que l'on relâche la pression sur l'actionneur latéral 53, le clapet de sortie 52 se ferme et le clapet d'entrée 51 s'ouvre sous l'effet de la dépression créée dans la chambre de pompe 50, permettant ainsi d'aspirer du produit fluide en provenance du réservoir 4, dont le piston suiveur 42 se déplace alors vers la pompe 5.

Comme on peut le comprendre à partir de la figure 1, le distributeur D est inséré à l'intérieur du corps creux 1 à travers son extrémité basse 17, après retrait du fond amovible 7. Le distributeur D est ainsi inséré axialement à travers l'espace 1a, puis à travers l'espace 1b jusqu'à ce que l'embout de distribution 6 pénètre à l'intérieur du logement 22 de la tête d'application 2. Comme précédemment expliqué, il est nécessaire d'orienter angulairement le distributeur D afin que son appendice d'insertion 63 s'engage à l'intérieur du logement 22. L'orientation angulaire est unique. On peut ainsi noter que la coopération de l'appendice d'insertion 63 avec le logement 22 forme des moyens d'orientation ou d'indexation angulaire, permettant d'orienter angulairement le distributeur D dans le corps de réception creux 1, de manière à ce que l'actionneur latéral 53 soit notamment disposé en face du poussoir 15 (ou de la fenêtre qui le remplacerait). Ces moyens d'orientation ou d'indexation angulaire nécessitent une recherche par tâtonnement de la part de l'utilisateur, qui doit faire tourner le distributeur D dans le corps creux 1 jusqu'à ce qu'il sente ou perçoive que l'appendice d'insertion 63 s'engage dans le logement 22.

Il est alors possible d'engager à fond l'appendice 63 à l'intérieur du logement 22 jusqu'à ce que la surface de sortie 61 parvienne au niveau de la surface d'application S de manière à la compléter. Ceci est visible sur la figure 2. On peut voir que la surface de sortie 61 vient parfaitement en affleurement avec la plage de surface d'application 21 de la tête 2 de manière à la compléter. Au final, seul l'orifice de distribution 62 rompt la continuité de la surface d'application S. Pour garantir l'engagement à fond de l'appendice 63 dans le logement 22, on se sert du fond amovible 7 dont la matière souple 64 vient en contact du fond du réservoir 4 pour le pousser vers le haut, et réaliser une étanchéité au niveau du logement 22. Il faut à cet égard également remarquer que l'extrémité inférieure du réservoir 4 fait saillie hors du corps creux 1 lorsque le fond amovible 7 est retiré, de manière à pouvoir aisément saisir le distributeur par son réservoir 4 pour l'extraire du corps creux 1. De ce fait, le distributeur D est reçu de manière amovible à l'intérieur du corps creux 1 et de la tête 2. On peut également remarquer que l'orientation angulaire imposée de l'appendice 63 à l'intérieur du logement 22 permet de disposer l'actionneur latéral 53 en face du poussoir 15 du corps creux 1.

Avec une telle conception, le distributeur D est reçu de manière amovible à l'intérieur de l'entité de réception R et à l'intérieur du logement 22 de l'entité d'application A. De cette manière, on peut remplacer à volonté le distributeur D et l'entité d'application A en fonction des besoins. On peut par exemple imaginer qu'un distributeur particulier distribuant un produit fluide particulier est associé à une entité d'application particulière.

Il faut aussi remarquer que le produit fluide distribué par le distributeur D ne sort du distributeur qu'au niveau de la surface d'application S, de sorte qu'il ne peut rester de produit fluide à l'intérieur de l'entité de réception R, une fois le distributeur retiré. De plus, du fait que la surface d'application S est parfaitement continue et lisse, elle est aisément nettoyable par frottement ou essuyage. Ainsi, lorsqu'un utilisateur veut changer de distributeur, il lui suffit au préalable de nettoyer la surface d'application S, puis de retirer le distributeur et de le remplacer par un autre. Aucune souillure ou dépôt de produit fluide ne peut être observé.

Bien que l'ensemble de distribution et d'application de produit fluide qui vient d'être décrit en référence aux figures 1 à 3 représente l'art antérieur, l'intégralité de ses caractéristiques et de son agencement, hormis au niveau des moyens d'orientation 22, 63, peut être reprise, sous forme identique ou modifiée, dans le cadre de la présente invention.

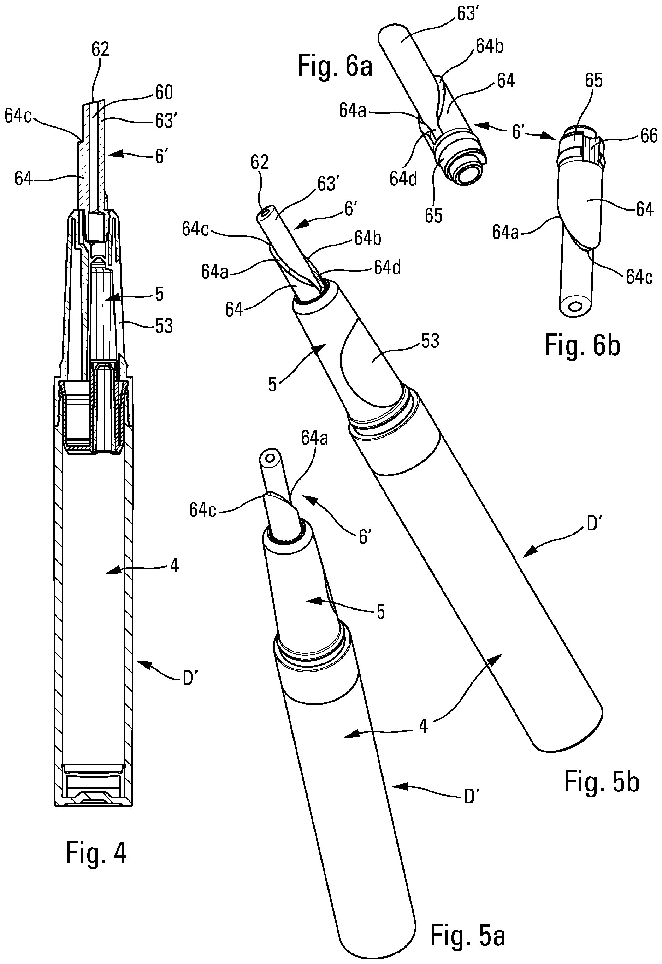

On se référera maintenant aux figures 4, 5a, 5b, 6a et 6b pour décrire un distributeur D' selon l'invention, qui ne diffère du distributeur D qu'au niveau de l'embout de distribution : le réservoir 4 et la pompe 5 peuvent être strictement identiques à ceux des figures 1 à 3. En revanche, l'embout de distribution 6' est différent. En effet, cet embout 6' comprend, en plus du talon d'ancrage 65 (qui peut être identique à celui de l'embout 6) et de l'appendice d'insertion 63' (qui différé de celui de l'embout 6), un profil d'orientation 64 qui est situé entre le talon d'ancrage 65 et l'appendice d'insertion 63'. Ce profil d'orientation 64 comprend, dans ce mode de réalisation particulier, deux rampes de came hélicoïdales 64a et 64b qui s'étendent de manière inclinée autour d'une extension vers le bas de l'appendice d'insertion 63'. Les deux rampes de came hélicoïdales 64a et 64b se rejoignent au niveau supérieur en formant une sorte de pointe 64c. Bien que cette pointe 64c soit quelque peu arrondie sur les figures, elle peut aussi être anguleuse ou acérée. Les deux rampes de came hélicoïdales 64a et 64b conduisent également toutes deux vers une encoche de butée 64d, qui est située juste au-dessus du talon d'ancrage 65. La pointe 64c et l'encoche de butée 64d sont ainsi situées de manière diamétralement opposée, mais décalée axialement, puisque la pointe 64c est adjacente ou proche de l'appendice d'insertion 63' et l'encoche de butée 64d est adjacente proche du talon d'ancrage 65. L'encoche de butée 64d est orientée de manière à être alignée avec l'actionneur latéral 53. Les deux rampes de came hélicoïdales 64a et 64b forment ainsi une sorte de collerette saillante inclinée qui entoure la base de l'appendice d'insertion 63'.

On peut noter que l'appendice d'insertion 63', ainsi que sa base située au niveau des deux rampes de came hélicoïdales, présente une section transversale ronde ou circulaire, contrairement à celle de l'appendice d'insertion 63 des figures 1 à 3. L'orientation du distributeur ne se fait donc plus au niveau de l'appendice d'insertion, mais au niveau des deux rampes de came hélicoïdales, de la pointe 64c et de l'encoche de butée 64d.

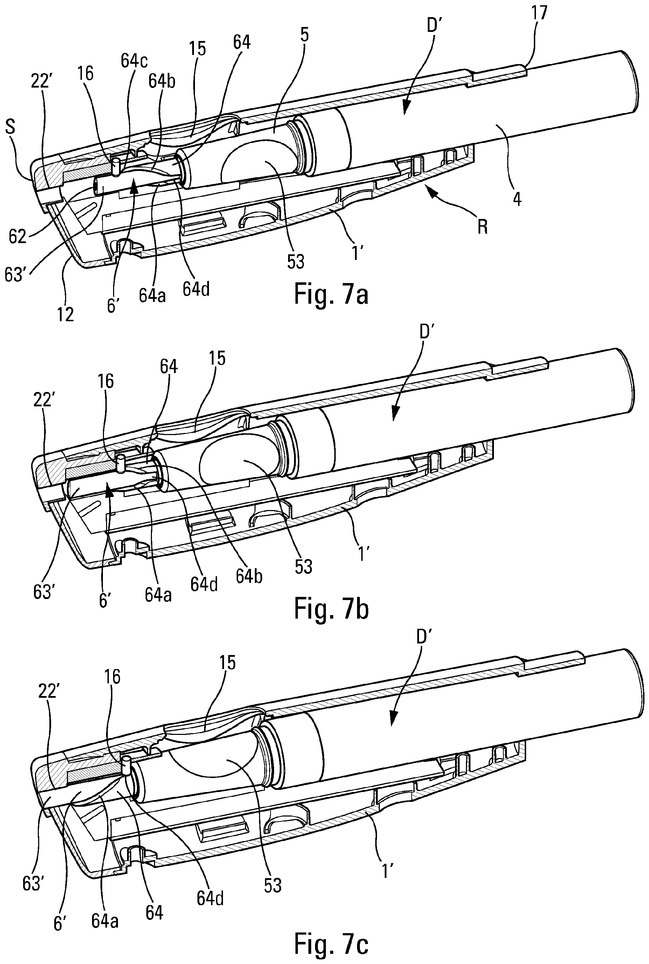

En se référant aux figures 7a, 7b et 7c, on peut voir de quelle manière le distributeur D' coopère avec le corps creux 1' de l'entité de réception R. Ces figures représentent l'ensemble de distribution et d'application de produit fluide de l'invention avec le distributeur D' en perspective, le corps creux en coupe transversale et l'entité d'application A retirée. On peut remarquer que le corps creux 1' comprend un ergot 16 qui est situé entre le logement 22' et le poussoir 15. Cet ergot 16 fait saillie radialement vers l'intérieur de manière à venir à proximité ou en contact avec l'appendice d'insertion 63', comme on peut le voir sur la figure 7a. Pour parvenir à cette position d'insertion, l'utilisateur a tout simplement introduit le distributeur D' dans le corps creux 1' à travers son extrémité inférieure ouverte (après retrait du fond 7). L'engagement du distributeur D' dans le corps creux 1' s'effectue axialement sans composante rotative, jusqu'à ce que l'ergot 16 vienne en contact avec le profil d'orientation 64. Selon l'orientation aléatoire du distributeur D' dans le corps creux 1', l'ergot va venir en contact soit avec la pointe 64c, soit avec l'une des rampes de came hélicoïdales 64a ou 64b, ou encore l'ergot 16 peut directement venir se loger dans l'encoche de butée 64d dans le meilleur des cas dans lequel le profil d'orientation 64 n'aurait pas d'utilité. Cependant, dans la plupart des cas, l'ergot 16 va venir en contact avec l'une des rampes de came hélicoïdales 64a ou 64b et va donc induire un mouvement de rotation du distributeur D' sur lui-même. L'ergot va coulisser le long de la rampe 64a ou 64b jusqu'à parvenir au regard de l'encoche de butée 64d. Le distributeur D' peut alors poursuivre son déplacement axial (sans composante rotative) jusqu'à ce que l'ergot arrive en butée en fond de l'encoche de butée 64d (figure 7c). Simultanément, l'appendice d'insertion 63' s'est engagé dans son logement 22'. L'appendice d'insertion 63' peut même s'engager dans son logement 22' alors que l'ergot 16 coulisse encore sur une des rampes, étant donné que la section transversale de l'appendice d'insertion 63' et de son logement 22' est ronde ou circulaire.

Sur la figure 7a, l'ergot 16 vient pratiquement en contact de la pointe 64c, mais du côté de la rampe 64b. Il va donc coulisser sur cette rampe 64b, comme représenté sur la figure 7b. Au final, il sa se loger dans l'encoche 64d, comme visible sur la figure 7c. L'actionneur latéral 53 est alors placé en face du poussoir 15 et l'appendice d'insertion 63' est engagé à fond dans son logement 22'. Le fond 7 peut alors être remis en place.

Les figures illustrent un mode de réalisation particulier, dans lequel le profil d'orientation est formé sur l'embout de distribution et l'ergot au niveau du corps creux. Sans sortir du cadre de l'invention, on peut aussi envisager les modes de réalisation alternatifs suivants :
- Le profil d'orientation est formé par le corps creux (ou est solidaire du corps creux), et l'ergot est formé par le distributeur.
- Le profil d'orientation ne comprend qu'une seule rampe de came qui forme une pointe et conduit à l'encoche de butée. Cette rampe unique s'étend alors sur près de 360°.
- La rampe de came est formée au niveau du logement 22.

Quel que soit le mode de réalisation, le profil d'orientation forme avec l'ergot (ou son équivalent) des moyens d'orientation ou d'indexation angulaire, permettant d'orienter angulairement le distributeur D' dans le corps de réception creux 1', de manière à ce que l'actionneur latéral 53 soit notamment disposé en face du poussoir 15 (ou de la fenêtre qui le remplacerait), et ce, sans que l'utilisateur n'est à rechercher une quelconque position angulaire particulière. En effet, quelle que soit l'orientation angulaire du distributeur D' lors de son engagement dans le corps creux, il va automatiquement s'orienter de manière adéquate, sans même que l'utilisateur ne s'en aperçoive ou en ait même conscience. L'orientation du distributeur D' peut se faire, au moins partiellement, lors de la mise en place du fond 7.

Grâce à l'invention, les moyens d'orientation angulaire induisent un mouvement de rotation du distributeur à mesure qu'il est poussé dans le corps de réception, jusqu'à ce qu'il atteigne sa position angulaire déterminée.

## Revendications

1. Ensemble de distribution et d'application de produit fluide comprenant :
- un corps de réception (1') creux, une surface d'application de produit fluide (S) étant située à une extrémité supérieure (12) du corps de réception (1') et destinée à venir en contact avec une cible, telle que la peau, cette surface d'application de produit fluide (S) comprenant un logement (22'),
- un distributeur de produit fluide (D') comprenant un réservoir (4), une pompe (5) et un embout de distribution (6') définissant un orifice de distribution de produit fluide (62), le distributeur (D') étant reçu de manière amovible dans le corps de réception (1') avec un appendice d'insertion (63') de son embout de distribution (6') inséré de manière amovible dans le logement (22'), de sorte que l'orifice de distribution (62) débouche directement dans la surface d'application (S), le distributeur (D') étant extractible hors du corps de réception (1') à travers une extrémité inférieure (17) ouverte du corps de réception (1') qui est opposée à l'extrémité supérieure (12), des moyens d'orientation angulaire (16, 64) étant prévus pour amener le distributeur de produit fluide (D') dans une position angulaire déterminée par rapport au corps de réception (1'),
**caractérisé en ce que** les moyens d'orientation angulaire (16, 64) induisent un mouvement de rotation du distributeur (D') à mesure qu'il est poussé dans le corps de réception (1'), jusqu'à ce qu'il atteigne sa position angulaire déterminée.

2. Ensemble selon la revendication 1, dans lequel les moyens d'orientation angulaire (16, 64) comprennent au moins une rampe de came hélicoïdale (64a, 64b).

3. Ensemble selon la revendication 2, dans lequel les moyens d'orientation angulaire (16, 64) comprennent un ergot (16) qui coulisse le long de la rampe de came hélicoïdale (64a, 64b) en induisant un mouvement de rotation au distributeur (D').

4. Ensemble selon l'une quelconque des revendications précédentes, dans lequel les moyens d'orientation angulaire (16, 64) comprennent deux rampes de came hélicoïdales (64a, 64b) qui se rejoignent au niveau d'une pointe (64c) et qui conduisent tous deux vers la position angulaire déterminée.

5. Ensemble selon l'une quelconque des revendications précédentes, dans lequel l'embout de distribution (6') forme, en amont de l'appendice d'insertion (63'), deux rampes de came hélicoïdales (64a, 64b) qui se rejoignent au niveau d'une pointe (64c) et qui conduisent tous deux vers une encoche de butée (64d), le corps de réception (1') formant un ergot fixe (16) qui coulisse le long d'une des deux rampes de came hélicoïdales (64a, 64b) jusqu'à venir se loger dans l'encoche de butée (64d).

6. Ensemble selon la revendication 5, comprenant un fond amovible (7) qui coopère avec le corps de réception (1') pour obturer l'extrémité inférieure (17), ce fond amovible (7) poussant le distributeur (D') vers l'extrémité supérieure (12) en appuyant l'ergot fixe (16) dans l'encoche de butée (64d).

7. Ensemble selon l'une quelconque des revendications précédentes, dans lequel le distributeur (D') définit un axe longitudinal X qui passe par le réservoir (4), la pompe (5) et l'embout de distribution (6'), la pompe (5) comprenant un actionneur latéral (53) qui est mobile transversalement à l'axe longitudinal X, le corps de réception (1') comprenant un poussoir latéral (15) qui est disposé en face de l'actionneur latéral (53) pour pourvoir déplacer l'actionneur latéral (53) en appuyant latéralement sur le poussoir latéral (15).

8. Ensemble selon l'une quelconque des revendications précédentes, dans lequel l'appendice d'insertion (63') présente une section transversale sensiblement circulaire.

9. Ensemble selon l'une quelconque des revendications précédentes, dans lequel l'embout de distribution (6') définit une surface de sortie (61) qui présente un contour extérieur dont la forme correspond à celle du logement (22') au moins au niveau de la surface d'application (S), de sorte que l'embout de distribution (6') obture le logement (22') de manière étanche, au moins au niveau de la surface d'application (S).

10. Ensemble selon l'une quelconque des revendications précédentes, dans lequel le corps de réception (1') abrite un module d'activation (3) apte à produire des ondes électromagnétiques telles que de la lumière, des vibrations et/ou des courants électriques tels que l'iontophorèse, au niveau de la surface d'application (S).

## Patentansprüche

1. Vorrichtung zur Abgabe und zum Auftragen eines Fluidprodukts, umfassend:
- einen hohlen Aufnahmekörper (1'), wobei sich eine Fluidprodukt-Auftragsfläche (S) an einem oberen Ende (12) des Aufnahmekörpers (1') befindet und dazu bestimmt ist, mit einem Ziel, wie der Haut, in Kontakt zu kommen, wobei diese Fluidprodukt-Auftragsfläche (S) ein Gehäuse (22') umfasst,
- einen Fluidproduktspender (D'), der einen Vorratsbehälter (4), eine Pumpe (5) und eine Ausgabespitze (6') umfasst, wobei letztere eine Fluidproduktausgabeöffnung (62) bildet, wobei der Spender (D') herausnehmbar in dem Aufnahmekörper (1') aufgenommen ist, wobei ein Einführfortsatz (63') seiner Ausgabespitze (6') herausnehmbar in das Gehäuse (22') eingesetzt ist, so dass die Abgabeöffnung (62) direkt in die Auftragsfläche (S) mündet, wobei der Spender (D') durch ein offenes unteres Ende (17) des Aufnahmekörpers (1'), das dem oberen Ende (12) gegenüberliegt, aus dem Aufnahmekörper (1') herausziehbar ist, wobei Winkelausrichtungsmittel (16, 64) vorgesehen sind, um den Fluidproduktspender (D') in eine bestimmte Winkelposition in Bezug auf den Aufnahmekörper (1') zu bringen,
**dadurch gekennzeichnet, dass** die Winkelausrichtungsmittel (16, 64) eine Drehbewegung des Spenders (D') bewirken, wenn dieser in den Aufnahmekörper (1') eingeschoben wird, bis er seine bestimmte Winkelposition erreicht.

2. Vorrichtung nach Anspruch 1, bei der die Winkelausrichtungsmittel (16, 64) mindestens eine schraubenförmige Nockenrampe (64a, 64b) umfassen.

3. Vorrichtung nach Anspruch 2, bei der die Winkelausrichtungsmittel (16, 64) einen Stift (16) umfassen, der an der schraubenförmigen Nockenrampe (64a, 64b) entlang gleitet und den Spender (D') in Drehbewegung versetzt.

4. Vorrichtung nach einem der vorhergehenden Ansprüche, bei der die Winkelausrichtungsmittel (16, 64) zwei schraubenförmige Nockenrampen (64a, 64b) umfassen, die sich in einer Spitze (64c) treffen und alle beide zu der bestimmten Winkelposition führen.

5. Vorrichtung nach einem der vorhergehenden Ansprüche, bei der die Ausgabespitze (6') stromaufwärts des Einführfortsatzes (63') zwei schraubenförmige Nockenrampen (64a, 64b) bildet, die sich in einer Spitze (64c) treffen und beide zu einer Anschlagkerbe (64d) führen, wobei der Aufnahmekörper (1') einen feststehenden Stift (16) ausbildet, der an einer der beiden schraubenförmigen Nockenrampen (64a, 64b) entlang gleitet, bis er in der Anschlagkerbe (64d) aufgenommen ist.

6. Vorrichtung nach Anspruch 5, mit einem abnehmbaren Boden (7), der mit dem Aufnahmekörper (1') zusammenwirkt, um das untere Ende (17) zu verschließen, wobei dieser abnehmbare Boden (7) den Spender (D') in Richtung des oberen Endes (12) drückt, indem er den feststehenden Stift (16) in die Anschlagkerbe (64d) drückt.

7. Vorrichtung nach einem der vorhergehenden Ansprüche, bei der der Spender (D') eine Längsachse X definiert, die durch den Vorratsbehälter (4), die Pumpe (5) und die Ausgabespitze (6') verläuft, wobei die Pumpe (5) ein seitliches Stellglied (53) aufweist, das quer zur Längsachse X bewegbar ist, der Aufnahmekörper (1') ein seitliches Schubglied (15) aufweist, das dem seitlichen Stellglied (53) gegenüberliegend angeordnet ist, um das seitliche Stellglied (53) durch seitliches Drücken des seitlichen Schubgliedes (15) bewegen zu können.

8. Vorrichtung nach einem der vorhergehenden Ansprüche, bei der der Einführfortsatz (63') einen im Wesentlichen kreisförmigen Querschnitt aufweist.

9. Vorrichtung nach einem der vorhergehenden Ansprüche, bei der die Ausgabespitze (6') eine Austrittsfläche (61) definiert, die in der Form ihrer Außenkontur der Form des Gehäuses (22') zumindest in Höhe der Auftragsfläche (S) entspricht, so dass die Ausgabespitze (6') das Gehäuse (22') zumindest in Höhe der Auftragsfläche (S) dicht abschließt.

10. Vorrichtung nach einem der vorhergehenden Ansprüche, bei der der Aufnahmekörper (1') ein Aktivierungsmodul (3) beherbergt, das in der Lage ist, elektromagnetische Wellen wie Licht, Vibrationen und/oder elektrische Ströme wie lontophorese an der Auftragsfläche (S) zu erzeugen.

## Claims

1. A fluid dispenser and applicator assembly comprising:
• a hollow reception body (1'), a fluid applicator surface (S) being situated at a top end (12) of the reception body (1') and being intended to come into contact with a target, such as the skin, the fluid applicator surface (S) including a housing (22'),
• a fluid dispenser (D') comprising a reservoir (4), a pump (5), and a dispenser endpiece (6') that defines a fluid dispenser orifice (62), the dispenser (D') being received in removable manner in the reception body (1') with an insertion appendage (63') of its dispenser endpiece (6') being inserted in removable manner in the housing (22'), so that the dispenser orifice (62) opens out directly in the applicator surface (S), the dispenser (D') being removable from the reception body (1') through an open bottom end (17) of the reception body (1') that is remote from the top end (12), angular orientation means (16, 64) being provided so as to bring the fluid dispenser (D') into a determined angular position relative to the reception body (1'),
the assembly being **characterized in that** the angular orientation means (16, 64) cause the dispenser (D') to turn as it is pushed into the reception body (1'), until it reaches its determined angular position.

2. An assembly according to claim 1, wherein the angular orientation means (16, 64) includes at least one helical cam-ramp (64a, 64b).

3. An assembly according to claim 2, wherein the angular orientation means (16, 64) include a lug (16) that slides along the helical cam-ramp (64a, 64b) causing the dispenser (D') to turn.

4. An assembly according to any preceding claim, wherein the angular orientation means (16, 64) include two helical cam-ramps (64a, 64b) that are joined together at a tip (64c) and that both lead to the determined angular position.

5. An assembly according to any preceding claim, wherein, upstream from the insertion appendage (63'), the dispenser endpiece (6') forms, two helical cam-ramps (64a, 64b) that are joined together at a tip (64c) and that both lead to an abutment notch (64d), the reception body (1') forming a stationary lug (16) that slides along one of the two helical cam-ramps (64a, 64b) until it becomes housed in the abutment notch (64d).

6. An assembly according to claim 5, including a removable bottom wall (7) that co-operates with the reception body (1') so as to close the bottom end (17), the removable bottom wall (7) acting to push the dispenser (D') towards the top end (12), pressing the stationary lug (16) into the abutment notch (64d).

7. An assembly according to any preceding claim, wherein the dispenser (D') defines a longitudinal axis X that passes via the reservoir (4), the pump (5), and the dispenser endpiece (6'), the pump (5) including a lateral actuator (53) that is movable transversally relative to the longitudinal axis X, the reception body (1') including a lateral pusher (15) that is arranged facing the lateral actuator (53) so as to make it possible to move the lateral actuator (53) by pressing laterally on the lateral pusher (15).

8. An assembly according to any preceding claim, wherein the insertion appendage (63') presents a cross-section that is substantially circular.

9. An assembly according to any preceding claim, wherein the dispenser endpiece (6') defines an outlet surface (61) that presents an outline having a shape that corresponds to the shape of the housing (22') at least at the applicator surface (S), such that the dispenser endpiece (6') closes the housing (22') in sealed manner, at least at the applicator surface (S).

10. An assembly according to any preceding claim, wherein the reception body (1') houses an activation module (3) that is suitable for producing electromagnetic waves such as light, vibration, and/or electricity such as iontophoresis, at the applicator surface (S).
